# EUROPÄISCHE PATENTSCHRIFT

(11) **EP 2 914 201 B1**
(45) Veröffentlichungstag und Bekanntmachung des Hinweises auf die Patenterteilung: **10.10.2018**
(21) Anmeldenummer: 13798257.5
(22) Anmeldetag: 30.10.2013
(51) Int. Cl.: A61C 9/00, G06F 19/00, G10L 21/02, G10L 21/04

(54) **VERFAHREN ZUR ERMITTLUNG MINDESTENS EINES RELEVANTEN EINZELBILDES EINES DENTALEN OBJEKTS**
METHOD FOR DETERMINING AT LEAST ONE RELEVANT SINGLE IMAGE OF A DENTAL SUBJECT
PROCÉDÉ PERMETTANT D'OBTENIR AU MOINS UNE IMAGE INDIVIDUELLE PERTINENTE D'UN OBJET DENTAIRE

(30) Priorität: 30.10.2012 DE 102012219865
(43) Veröffentlichungstag der Anmeldung: 09.09.2015
(73) Patentinhaber: Sirona Dental Systems GmbH, 64625 Bensheim (DE)
(72) Erfinder: SCHMITT, Bernhard, 67067 Ludwigshafen (DE)
(74) Vertreter: Sommer, Peter
(86) Internationale Anmeldenummer: PCT/EP2013/072669
(87) Internationale Veröffentlichungsnummer: WO 2014/067977

(56) Entgegenhaltungen:
- EP-A2- 0 784 965
- EP-A2- 1 252 859
- JP-A- 2005 097 277
- US-A1- 2006 256 193
- US-A1- 2007 026 363

## Beschreibung

### Technisches Gebiet

Die Erfindung betrifft ein Verfahren zur Ermittlung mindestens eines relevanten Einzelbildes, wobei mehrere optische Einzelbilder während einer kontinuierlichen optischen Vermessung eines aufzunehmenden dentalen Objekts erzeugt werden.

### Stand der Technik

Aus dem Stand der Technik sind bereits Verfahren zur Archivierung von zahnärztlichen Befunden bekannt. Dabei untersucht der Zahnarzt einen Patienten und kommuniziert den Status und den Befund an eine zahnmedizinische Fachangestellte, die den Status oder Befund meist manuell in eine Patientenakte einträgt. Diese Patientenakte kann beispielsweise auch in einer besonderen Software angelegt sein.

DE 199 16 162 A1 offenbart ein Dentallasersystem bestehend aus mehreren Modulen, wobei das Dentallasersystem eine Leseeinheit, ein Multimediamodul, ein Scannermodul, eine Protokolliereinheit und ein Sprachsteuerungsmodul aufweist. Das Sprachsteuerungsmodul kann ein kleines Mikrophon aufweisen, das beispielsweise in eine dentale Kamera integriert sein kann. Mittels des Sprachsteuerungsmoduls können Kommentare zu den einzelnen aufgenommenen Bildern hinzugefügt werden, die anschließend archiviert und wieder aufgerufen werden können. Der behandelnde Arzt kann das Behandlungsergebnis auch mittels des Sprachsteuerungsmoduls eingeben, wobei mittels eines Sprachübersetzungsprogramms die Eingabe des Behandlungsergebnisses erfolgt. Anschließend können die Behandlungsergebnisse in Karteikarten übernommen werden.

US20060256193 offenbart ein Verfahren wie im Oberbegriff des Anspruchs 1 definiert.

DE 10 2007 054 907 A1 offenbart ein Verfahren zur optischen Vermessung eines Objekts unter Verwendung eines Triangulationsverfahrens mittels einer optischen Aufnahmevorrichtung, wobei mehrere Lichtquellen und mehrere Blendenmittel verwendet werden, um mehrere Muster gleichzeitig auf das Objekt zu projizieren. Zur Vermessung kann vorteilhafterweise ein Streifenprojektionsverfahren verwendet werden, wobei ein Muster aus parallelen Streifen auf das Objekt projiziert wird und dadurch mehrere Messpunkte gleichzeitig vermessen werden.

Darüber hinaus sind Verfahren zur Archivierung bekannt, bei denen zusätzlich Videoaufnahmen des betreffenden Gebiets im Mundraum des Patienten aufgenommen werden und dem jeweiligen Status oder Befund manuell zugeordnet werden. Dabei wird die Videoaufnahme meist durch das Betätigen eines Schalters an einer dentalen Kamera oder eines Fußschalters ausgelöst.

Ein Nachteil dieses Verfahrens besteht darin, dass es durch eine Unachtsamkeit der zahnmedizinischen Fachangestellten zu einer fehlerhaften Zuordnung der Befunde kommen kann. Dies kann zu einem Behandlungsfehler führen oder eine erneute Untersuchung des Patienten notwendig machen.

Die Aufgabe der vorliegenden Erfindung besteht daher darin, ein Verfahren bereitzustellen, das eine sichere und zeitsparende Zuordnung von Einzelbildern ermöglicht, die mit wenig Aufwand verbunden ist.

### Darstellung der Erfindung

Die Erfindung betrifft ein Verfahren zur Ermittlung mindestens eines relevanten Einzelbildes, wobei mehrere optische Einzelbilder während einer kontinuierlichen optischen Vermessung eines aufzunehmenden dentalen Objekts erzeugt werden. Während der optischen Vermessung wird mittels eines Mikrofons eine Audioaufnahme erzeugt und dabei mindestens eine Sprachsequenz aufgezeichnet, die durch einen Benutzer ausgesprochen wird. Anschließend wird innerhalb einer bestimmten Sequenzdauer zwischen einem Anfangszeitpunkt und einem Endzeitpunkt der Sprachsequenz das relevante Einzelbild ausgewählt. Innerhalb der ermittelten Sprachsequenz wird dasjenige relevante Einzelbild ausgewählt, das zu einem Zeitpunkt aufgenommen wurde, zu dem eine Bewegung der dentalen Kamera relativ zum aufzunehmenden Objekt am geringsten war.

Dadurch wird das relevante Einzelbild mit der geringsten Verwacklung der Kamera relativ zum Objekt ausgewählt. Ein solches Einzelbild weist die geringsten Bewegungsartefakte und damit die beste Aufnahmequalität aller Einzelbilder der jeweiligen Sprachsequenz auf.

Die optische Vermessung kann beispielsweise mittels einer Videoaufnahme, bei der mehrere einzelne Videobilder aufgenommen werden, oder mittels eines optischen Streifenprojektionsverfahrens erfolgen. Bei dem Streifenprojektionsverfahren wird ein Streifenmuster auf das zu vermessende Objekt projiziert und aufgrund der optischen Verzerrung des Streifenmusters die dreidimensionale Oberfläche des Objekts ermittelt. Auf diese Weise wird das gesamte Objekt kontinuierlich in mehreren 3D-Bildern aufgenommen. Das dentale Objekt kann beispielsweise die gesamte Zahnsituation des Patienten oder ein Teil davon, wie ein Oberkiefer, ein Unterkiefer, eine Präparation oder auch einzelne Zähne sein. Das Mikrofon für die Audioaufnahme kann beispielsweise direkt in die dentale Kamera integriert sein. Alternativ dazu kann das Mikrofon auch in ein Headset integriert sein, das sich der Benutzer während der optischen Vermessung aufsetzt oder in eine dentale Behandlungseinheit integriert sein. Der Benutzer, wie ein behandelnder Zahnarzt oder auch ein zahnmedizinischer Fachangestellter, spricht dabei während der optischen Vermessung des dentalen Objekts Statusinformationen und/oder Befundinformationen über den jeweils untersuchten Bereich des Objekts aus. Diese Sprachsequenzen werden daraufhin aufgezeichnet und ausgewertet. Die Sprachsequenz kann dabei mittels einer herkömmlichen Videokamera oder mittels eines Mikrofons aufgezeichnet werden. Innerhalb der Sequenzdauer der Sprachsequenz wird daraufhin das relevante Einzelbild ausgewählt und der jeweiligen Sprachsequenz zugeordnet. Die Auswahl des relevanten Einzelbildes kann aufgrund einer geringen lokalen Verwacklung erfolgen.

Ein Vorteil dieses Verfahrens besteht darin, dass das relevante Einzelbild automatisch der jeweiligen Sprachsequenz zugeordnet wird. Dadurch werden Verwechslungen, die zu Behandlungsfehlern führen können, verhindert.

Ein weiterer Vorteil dieses Verfahrens besteht darin, dass der Benutzer, wie ein behandelnder Zahnarzt, die Diagnose selbstständig ohne einen zahnmedizinischen Fachangestellten durchführen kann. Dies verkürzt die Dauer der Diagnose und ist weniger fehleranfällig.

Vorteilhafterweise können bei der Verarbeitung der Audioaufnahme Hintergrundgeräusche mittels einer Filtereinheit herausgefiltert werden, um die Audioqualität der Sprachsequenz zu verbessern.

Als Filtereinheit könnte beispielsweise ein sogenanntes Noise-Gate verwendet werden, das Sprachsignale verstärkt und das Hintergrundrauschen herausfiltert. Dadurch wird der Dynamikumfang des Audiosystems verändert, der durch das Grundrauschen des Audiosystems und die maximale Aussteuerung ohne hörbare Verzerrungen begrenzt ist. Dadurch wird die Audioqualität der aufgenommenen Sprachsequenzen verbessert, um die genaue Sequenzdauer zu bestimmen.

Zusätzlich können die aufgenommenen Sprachsequenzen mittels einer Spracherkennungssoftware von Sprache in Text übertragen werden. Dabei ist die Verwendung der Filtereinheit ebenfalls hilfreich.

Vorteilhafterweise kann die Filtereinheit innerhalb einer vorher festgelegten variablen Zeitdauer aktiviert bleiben.

Die Filtereinheit wird also erst nach dem Endzeitpunkt der Sprachsequenz aktiviert, und zwar für eine festgelegte Zeitdauer, auch Release-Zeit genannt.

Alternativ dazu kann die Filtereinheit auch für die gesamte Sequenzdauer aktiviert bleiben.

Bei einer weiteren Alternative kann die Filtereinheit während der gesamten optischen Vermessung aktiviert bleiben.

Vorteilhafterweise kann eine maximale Sequenzdauer und/oder eine minimale Sequenzdauer der Sprachsequenz vor der Durchführung des Verfahrens festgelegt werden.

Dadurch wird das relevante Einzelbild innerhalb der vorher festgelegten maximalen Sequenzdauer nach dem Anfangszeitpunkt der Sprachsequenz ausgewählt, auch wenn der Benutzer eine längere Zeit redet. Auf diese Weise wird ein Einzelbild eines Bereichs einer Zahnsituation ausgewählt, das der jeweiligen Sprachsequenz und der darin enthaltenen Befundinformation entspricht. Die festgelegte maximale Sequenzdauer kann beispielsweise zwei Sekunden betragen.

Vorteilhafterweise kann die Sequenzdauer der Sprachsequenz der Dauer eines zusammenhängenden Redeflusses entsprechen, wobei kurze Unterbrechungen des Redeflusses ignoriert werden, die kürzer als eine festgelegte Unterbrechungsdauer sind.

Dadurch wird der zusammenhängende Redefluss trotz kurzer Unterbrechungen als eine einzige Sprachsequenz erkannt. Dadurch werden bei kurzen Unterbrechungen also keine neuen Sprachsequenzen gestartet, sodass für den zusammenhängenden Redefluss mit Unterbrechungen jeweils nur ein einzelnes Einzelbild ausgesucht wird und diesem zugeordnet wird.

Vorteilhafterweise kann die Unterbrechungsdauer maximal 2 Sekunden betragen.

Auf diese Weise führen kurze Unterbrechungen des Sprachflusses bis zu zwei Sekunden nicht zu einem Abbruch der Sprachsequenz.

Vorteilhafterweise kann innerhalb einer Sprachsequenz nur ein relevantes Einzelbild ausgewählt werden.

Dadurch wird jeder Sprachsequenz nur ein einzelnes relevantes Einzelbild zugeordnet, so dass für den behandelnden Zahnarzt klar ist, welche Befundinformation und/oder Statusinformation zu welchem Bereich der aufgenommenen Zahnsituation gehört.

Vorteilhafterweise kann die mindestens eine Sprachsequenz eine Statusinformation und/oder eine Befundinformation enthalten, wobei die Statusinformation und/oder die Befundinformation mittels einer Spracherkennungssoftware von Sprache in Text übersetzt werden.

Dadurch werden die in der Sprachsequenz enthaltenen Statusinformationen und/oder Befundinformationen von Sprache in Text übersetzt und können in Textform zusammen mit den relevanten Einzelbildern angezeigt werden. Dadurch bekommt der behandelnde Zahnarzt einen umfassenden Überblick über die Gesamtsituation des dentalen Objekts.

Die Sprachsequenz kann zusätzlich eine anatomische Positionsangabe, wie "Zahn 11 lingual", enthalten, die mittels der Spracherkennungssoftware von Sprache in Text übersetzt werden kann. Diese anatomische Positionsangabe wird dann zusammen mit der Status- und/oder Befundinformation der jeweiligen Sprachsequenz zugeordnet. Der behandelnde Arzt kann auf diese Weise die einzelnen Zähne der Reihe nach diagnostizieren und die anatomische Positionsangabe mit der jeweiligen Befundinformation diktieren, wie "Zahn 18 labial", "kariös" "Zahn 17 distal", "eine notwendige Füllung". Auf diese Weise werden Fehler bei der Diagnose durch falsche Zuordnung der Befundinformationen verhindert.

Vorteilhafterweise kann die erkannte Statusinformation und/oder die Befundinformation in Textform dem relevanten Einzelbild der jeweiligen Sprachsequenz zugeordnet werden.

Dadurch ist es für den behandelnden Zahnarzt gleich ersichtlich, zu welchem Einzelbild welche Statusinformation und/oder welche Befundinformation gehört.

Eine Befundinformation kann beispielsweise ein mit Karies befallener Zahn, ein fehlender Zahn, ein ersetzter Zahn, ein Implantat mit einer intakten Krone, ein zu entfernendes Implantat, eine klinisch intakte Krone, ein erhaltungswürdiger Zahn mit partiellen Substanzdefekten, eine erneuerungsbedürftige Wurzelstiftkappe, eine unzureichende Retention, ein nicht erhaltungswürdiger Zahn, ein Lückenschluss, eine notwendige Krone, ein einzusetzendes Brückenglied, eine einzusetzende Teilkrone oder eine einzusetzende Verblendung sein.

Vorteilhafterweise können während der optischen Vermessung des gesamten Objekts mehrere relevante Einzelbilder zu den jeweiligen Sprachsequenzen ausgewählt werden, wobei die einzelnen relevanten Einzelbilder mittels einer Anzeigevorrichtung angezeigt werden.

Dadurch können die Einzelbilder zu den jeweiligen Sprachsequenzen gleichzeitig mittels der Anzeigevorrichtung, wie eines Monitors, angezeigt werden. Der behandelnde Zahnarzt bekommt dadurch einen schnellen Überblick über die Gesamtsituation des aufgenommenen dentalen Objekts, wie eines Oberkiefers oder eines Unterkiefers.

Vorteilhafterweise können die Sprachsequenzen anatomische Positionsangaben umfassen, die mittels einer Spracherkennungssoftware von Sprache in Text übertragen werden, wobei die Positionen der relevanten Einzelbilder relativ zum aufgenommenen Objekt unter Verwendung der anatomischen Positionsangaben ermittelt werden. Anschließend können die relevanten Einzelbilder an den ermittelten Positionen relativ zu einem virtuellen Modell des Objekts mittels der Anzeigevorrichtung dargestellt werden.

Der Benutzer, wie ein behandelnder Arzt, diktiert also während der Diagnose die anatomischen Positionsangaben mit den jeweiligen Befundinformationen, beispielsweise wie folgt: "Zahn 18 labial", "kariös" "Zahn 17 distal", "eine notwendige Füllung". Mittels der Spracherkennungssoftware werden dann die anatomischen Positionsangaben "Zahn 18 labial", "Zahn 17 distal" erkannt. Anschließend werden die Befundinformationen "kariös", "eine notwendige Füllung" den jeweiligen Positionen zugeordnet und am virtuellen Modell des Objekts dargestellt.

Alternativ dazu können die Positionen der relevanten Einzelbilder relativ zum aufgenommenen Objekt unter Verwendung eines Mustererkennungsverfahrens ermittelt werden.

Dadurch werden die Einzelbilder in Relation zum virtuellen Modell des aufgenommenen Objekts dargestellt, um die Orientierung zu erleichtern. Der Benutzer kann das virtuelle Modell auch drehen und aus unterschiedlichen Richtungen anschauen, um die Befundinformationen besser zu beurteilen.

Vorteilhafterweise können die ausgewählten relevanten Einzelbilder in mehrere Kategorien von Statusinformationen und/oder Befundinformationen sortiert werden.

Dadurch können die Befundinformationen, sortiert nach mehreren Kategorien, dargestellt werden, um dem behandelnden Zahnarzt einen einfacheren Überblick über die Befundinformationen und damit eine einfachere Behandlungsplanung zu ermöglichen. Die Befundinformationen können beispielsweise in Kategorien wie befallenes Zahngewebe, zu entfernende Zahnersatzteile und einzusetzende Zahnersatzteile eingeteilt werden.

Vorteilhafterweise kann die optische Vermessung des Objekts mittels einer Videoaufnahme erfolgen und einzelne Videobilder dieser Videoaufnahme die ausgewählten Einzelbilder sein, so dass zu jeder Sprachsequenz ein relevantes Videobild ausgewählt wird.

Dadurch erfolgt die optische Vermessung mittels der Videoaufnahme unter Verwendung einer herkömmlichen Videokamera, die in ein herkömmliches dentales Handstück integriert ist.

Vorteilhafterweise kann die optische Vermessung des Objekts mittels eines optischen dreidimensionalen Streifenprojektionsverfahrens erfolgen, wobei mehrere einzelne 3D-Bilder des Objekts erzeugt werden und zu einer dreidimensionalen Gesamtaufnahme zusammengefügt werden. Diese einzelnen 3D-Bilder stellen damit die auszuwählenden Einzelbilder dar, so dass zu jeder Sprachsequenz ein relevantes 3D-Bild des Objekts ausgewählt wird.

Dadurch erfolgt die optische Vermessung des dentalen Objekts mittels einer dentalen Kamera, die auf dem Streifenprojektionsverfahren beruht. Diese Vermessung ermöglicht die Erstellung eines dreidimensionalen Gesamtbildes des dentalen Objekts.

### Kurzbeschreibung der Zeichnungen

Die Erfindung wird anhand der Zeichnungen erläutert. Es zeigt, die
- Fig. 1: eine Skizze zur Verdeutlichung des Verfahrens;
- Fig. 2: ein Diagramm zur Verdeutlichung des zeitlichen Ablaufs des Verfahrens;
- Fig. 3: einen Computer mit einer Anzeigevorrichtung zur Darstellung der ausgewählten Einzelbilder.

### Ausführungsbeispiel

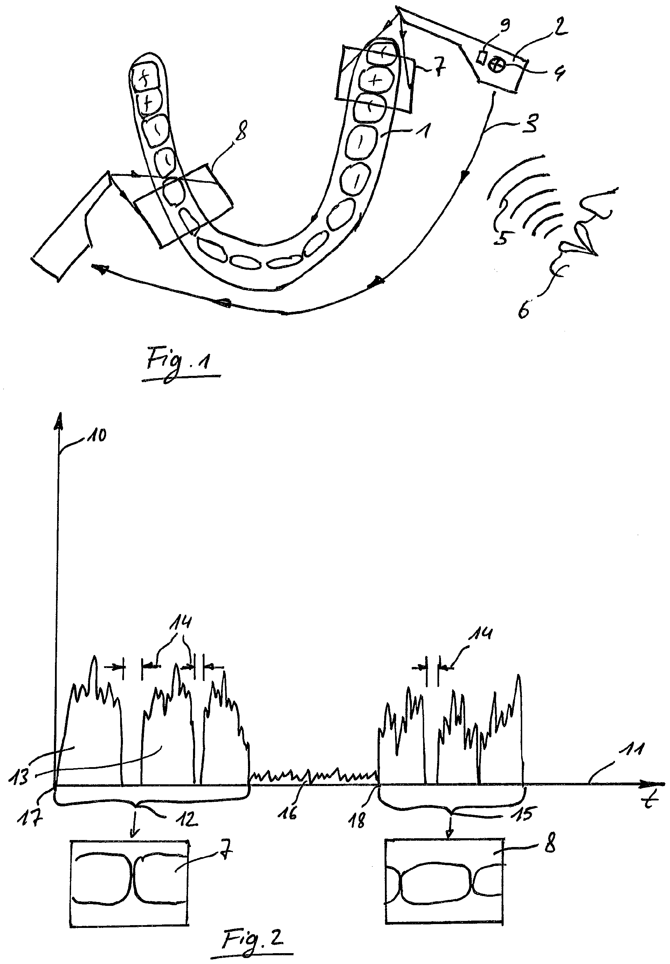

Die Fig. 1 zeigt eine Skizze zur Verdeutlichung des Verfahrens zur Ermittlung von relevanten Einzelbildern. Ein dentales Objekt 1, wie ein Unterkiefer, wird mittels einer optischen dentalen Kamera 2 vollständig vermessen, wobei die handgehaltene Kamera 2 um das dentale Objekt 1 entlang einer Bewegungsrichtung 3 geführt wird, die durch die Pfeile dargestellt ist. Während der optischen Vermessung werden kontinuierlich Einzelbilder von verschiedenen Abschnitten des Objekts 1 aufgenommen. Während der optischen Vermessung wird mittels eines Mikrofons 4, das in die Kamera 2 integriert ist, eine Audioaufnahme aufgenommen. Dabei wird mindestens eine Sprachsequenz 5 aufgezeichnet, die durch einen Benutzer 6, wie einen behandelnden Zahnarzt oder einen zahnmedizinischen Fachangestellten, ausgesprochen wird. Innerhalb einer Sequenzdauer der ersten Sprachsequenz 5 wird dann ein erstes relevantes Einzelbild 7 und später während der Sequenzdauer einer zweiten Sprachsequenz wird ein zweites relevantes Einzelbild 8 ausgewählt. Die Auswahl der relevanten Einzelbilder 7 und 8 kann aufgrund des Kriteriums der geringsten Verwacklung erfolgen. Dabei wird die Bewegung der Kamera 2 in Relation zum dentalen Objekt 1 bestimmt und ein relevantes Einzelbild in einem lokalen Minimum dieser relativen Bewegung ausgewählt. Ein solches Einzelbild weist dadurch die geringsten Bewegungsartefakte auf. Zur besseren Verarbeitung der Audioaufnahme ist in die Kamera 2 eine Filtereinheit 9 integriert, die die Sprachsignale der Sprachsequenz 5 verstärkt und die Hintergrundgeräusche herausfiltert.

Die Fig. 2 zeigt ein Diagramm zur Verdeutlichung des Ablaufs des Verfahrens. Der Betrag einer Amplitude 10 des aufgenommenen Audiosignals ist dabei auf der Zeitachse 11 aufgetragen. Eine erste Sprachsequenz 12 besteht dabei aus mehreren Teilen 13, die einen zusammenhängenden Redefluss mit kurzen Unterbrechungen 14 bilden. Diese Unterbrechungen 14 sind jedoch kürzer als eine festgelegte Unterbrechungsdauer von beispielsweise 2 Sekunden und werden ignoriert. Somit wird eine neue Sprachsequenz erst dann angefangen, wenn die Unterbrechung die festgelegte Unterbrechungsdauer überschreitet. Innerhalb der Sequenzdauer der ersten Sprachsequenz 12 wird das erste relevante Einzelbild 7 ausgewählt und dieser ersten Sprachsequenz zugeordnet. Später wird eine zweite Sprachsequenz 15 aufgenommen, die ebenfalls mehrere Teile mit einer kurzen Unterbrechung 14 aufweist. Innerhalb der Sequenzdauer der zweiten Sprachsequenz 15 wird dann das zweite relevante Einzelbild 8 ausgewählt und dieser zweiten Sprachsequenz 15 zugeordnet. Zwischen der ersten Sprachsequenz 12 und der zweiten Sprachsequenz 15 werden mittels des Mikrofons 4 Hintergrundgeräusche 16 aufgenommen, die jedoch mittels der Filtereinheit 9 herausgefiltert werden.

Alternativ dazu kann das relevante Einzelbild auch innerhalb einer festgelegten Zeitdauer von beispielsweise 2 Sekunden nach einem ersten Anfangspunkt 17 der ersten Sprachsequenz 12 und nach einem zweiten Anfangszeitpunkt 18 der zweiten Sprachsequenz 15 ausgewählt werden, um Einzelbilder aus längeren Sprachsequenzen zu detektieren.

Die erste Sprachsequenz 12 und die zweite Sprachsequenz 15 können dabei Statusinformationen und/oder Befundinformationen enthalten, die mittels einer Spracherkennungssoftware von Sprache in Text übertragen werden können. Der detektierte Text kann anatomische Positionsangaben beinhalten, die extrahiert und zur Darstellung verwendet werden können.

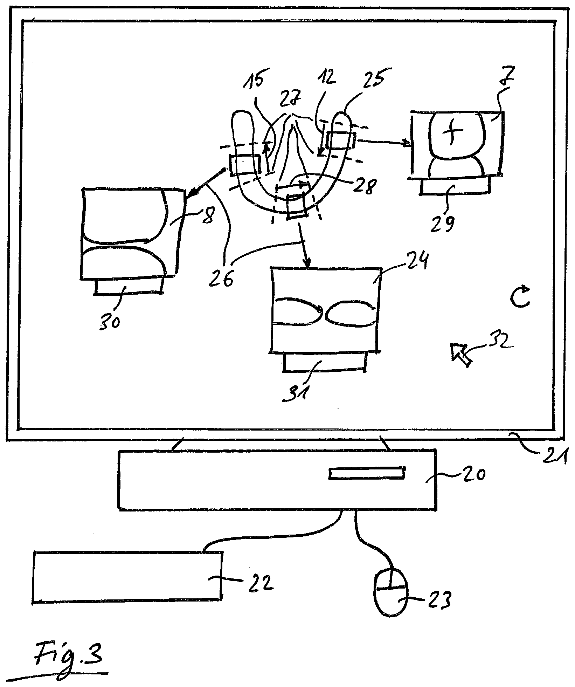

Die Fig. 3 zeigt einen Computer 20 mit einer Anzeigevorrichtung 21, wie einem Monitor, sowie mit Eingabegeräten, wie einer Tastatur 22 und einer Maus 23. Das erste Einzelbild 7, das zweite Einzelbild 8 und das dritte Einzelbild 24 werden mittels der Anzeigevorrichtung 21 an einem virtuellen Modell 25 des aufzunehmenden Objekts 1 in einer Explosionsansicht vergrößert dargestellt. Die Pfeile 26 zeigen die genaue Position der ausgesuchten Einzelbilder 7, 8 und 24 relativ zum Modell 25 des Objekts 1. Durch die gestrichelten Linien 27 werden die Anfangszeitpunkte und die Endzeitpunkte der ersten Sprachsequenz 12, der zweiten Sprachsequenz 15 und einer dritten Sprachsequenz 28 angezeigt, wobei die Bewegungsrichtung während der Aufnahme der Sprachsequenzen 12, 15 und 28 durch die Pfeile dargestellt ist. Darüber hinaus werden eine erste Befundinformation in Textform in einem Kästchen unterhalb des ersten Einzelbildes 7, eine zweite Befundinformation 30 unterhalb des zweiten Einzelbildes 8 und eine dritte Befundinformation unterhalb des dritten Einzelbildes 24 dargestellt. Auf diese Weise erhält der Benutzer, wie ein behandelnder Zahnarzt, einen schnellen Überblick über die Zahnsituation und die archivierten Befunde. Mittels der Eingabemittel 22 und 23 kann der Benutzer über einen Cursor 32 die Blickrichtung auf das Modell 25 durch das Drehen des Modells ändern.

### Bezugszeichen

- 1: dentales Objekt
- 2: dentale Kamera
- 3: Bewegungsrichtung
- 4: Mikrofon
- 5: Sprachsequenz
- 6: Benutzer
- 7: erstes relevantes Einzelbild
- 8: zweites relevantes Einzelbild
- 9: Filtereinheit
- 10: Amplitude
- 11: Zeitachse
- 12: erste Sprachsequenz
- 13: mehrere Teile einer Sprachsequenz
- 14: kurze Unterbrechung
- 15: zweite Sprachsequenz
- 16: Hintergrundgeräusche
- 17: Anfangszeitpunkt der ersten Sprachsequenz
- 18: Anfangszeitpunkt der zweiten Sprachsequenz
- 20: Computer
- 21: Anzeigevorrichtung
- 22: Tastatur
- 23: Maus
- 24: drittes relevantes Einzelbild
- 25: Modell des Objekts
- 26: genaue Position der Einzelbilder
- 27: Anfangs- und Endzeitpunkte der Sprachsequenzen
- 28: dritte Sprachsequenz
- 29: erste Befundinformation
- 30: zweite Befundinformation
- 31: dritte Befundinformation
- 32: Cursor

## Patentansprüche

1. Verfahren zur Ermittlung mindestens eines relevanten Einzelbildes, wobei mehrere optische Einzelbilder während einer kontinuierlichen optischen Vermessung (3) eines aufzunehmenden dentalen Objekts (1) erzeugt werden, wobei während der optischen Vermessung (3) mittels eines Mikrofons (4) eine Audioaufnahme erzeugt wird und dabei mindestens eine Sprachsequenz (12; 15; 28) aufgezeichnet wird, die durch einen Benutzer (6) ausgesprochen wird, **dadurch gekennzeichnet, dass** innerhalb einer bestimmten Sequenzdauer zwischen einem Anfangszeitpunkt (17, 18) und einem Endzeitpunkt der Sprachsequenz (12; 15; 28) das relevante Einzelbild (7; 8; 24) ausgewählt wird, wobei innerhalb der ermittelten Sprachsequenz das relevante Einzelbild (7; 8; 24) ausgewählt wird, das zu einem Zeitpunkt aufgenommen wurde, zu dem eine Bewegung (3) der dentalen Kamera (2) relativ zum aufzunehmenden Objekt (1) am geringsten war.

2. Verfahren nach Anspruch 1, **dadurch gekennzeichnet, dass** bei der Verarbeitung der Audioaufnahme Hintergrundgeräusche (16) mittels einer Filtereinheit (9) herausgefiltert werden, um die Audioqualität der Sprachsequenz (12; 15; 28) zu verbessern.

3. Verfahren nach Anspruch 2, **dadurch gekennzeichnet, dass** die Filtereinheit (9) innerhalb einer vorher festgelegten variablen Zeitdauer aktiviert bleibt.

4. Verfahren nach einem der Ansprüche 1 bis 3, **dadurch gekennzeichnet, dass** eine maximale Sequenzdauer (12; 15; 28) und/oder eine minimale Sequenzdauer der Sprachsequenz vor der Durchführung des Verfahrens festgelegt wird.

5. Verfahren nach einem der Ansprüche 1 bis 3, **dadurch gekennzeichnet, dass** die Sequenzdauer der Sprachsequenz (12; 15; 28) der Dauer eines zusammenhängenden Redeflusses (13) entspricht, wobei kurze Unterbrechungen (14) des Redeflusses ignoriert werden, die kürzer als eine festgelegte Unterbrechungsdauer sind.

6. Verfahren nach Anspruch 5, **dadurch gekennzeichnet, dass** die Unterbrechungsdauer maximal 2 Sekunden beträgt.

7. Verfahren nach Anspruch 6, **dadurch gekennzeichnet, dass** innerhalb einer Sprachsequenz (12; 15; 28) nur ein relevantes Einzelbild (7; 8; 24) ausgewählt wird.

8. Verfahren nach einem der Ansprüche 1 bis 7, **dadurch gekennzeichnet, dass** die mindestens eine Sprachsequenz (12; 15; 28) eine Statusinformation und/oder eine Befundinformation (29; 30; 31) enthält, wobei die Statusinformation und/oder die Befundinformation (29; 30; 31) mittels einer Spracherkennungssoftware von Sprache in Text übertragen werden.

9. Verfahren nach Anspruch 1, **dadurch gekennzeichnet, dass** die erkannte Statusinformation und/oder die Befundinformation (29; 30; 31) in Textform dem relevanten Einzelbild (7; 8; 24) der jeweiligen Sprachsequenz (12; 15; 28) zugeordnet wird.

10. Verfahren nach einem der Ansprüche 1 bis 9, **dadurch gekennzeichnet, dass** während der optischen Vermessung des gesamten Objekts (1) mehrere relevante Einzelbilder (7; 8; 24) zu den jeweiligen Sprachsequenzen (12; 15; 28) ausgewählt werden, wobei die einzelnen relevanten Einzelbilder (7; 8; 24) mittels einer Anzeigevorrichtung (21) angezeigt werden.

11. Verfahren nach Anspruch 10, **dadurch gekennzeichnet, dass** die Sprachsequenzen (12; 15; 28) anatomische Positionsangaben umfassen, die mittels einer Spracherkennungssoftware von Sprache in Text übertragen werden, wobei Positionen der relevanten Einzelbilder (7; 8; 24) relativ zum aufgenommenen Objekt (1) unter Verwendung der anatomischen Positionsangaben ermittelt werden, wobei die relevanten Einzelbilder (7; 8; 24) an den ermittelten Positionen relativ zu einem virtuellen Modell (25) des Objekts (1) mittels der Anzeigevorrichtung (21) dargestellt werden.

12. Verfahren nach einem der Ansprüche 1 bis 11, **dadurch gekennzeichnet, dass** die ausgewählten relevanten Einzelbilder (7; 8; 24) in mehrere Kategorien von Statusinformationen und/oder Befundinformationen (29; 30; 31) sortiert werden.

13. Verfahren nach einem der Ansprüche 1 bis 12, **dadurch gekennzeichnet, dass** die optische Vermessung des Objekts (1) mittels einer Videoaufnahme erfolgt und einzelne Videobilder dieser Videoaufnahme die ausgewählten Einzelbilder (7; 8; 24) sind, so dass zu jeder Sprachsequenz (12; 15; 28) ein relevantes Videobild (7; 8; 24) ausgewählt wird.

14. Verfahren nach einem der Ansprüche 1 bis 12, **dadurch kennzeichnet, dass** die optische Vermessung des Objekts (1) mittels eines optischen dreidimensionalen Streifenprojektionsverfahrens erfolgt, wobei mehrere einzelne 3D-Bilder des Objekts (1) erzeugt werden und zu einer dreidimensionalen Gesamtaufnahme zusammengefügt werden, wobei diese einzelnen 3D-Bilder die auszuwählenden Einzelbilder darstellen, so dass zu jeder Sprachsequenz (12; 15; 28) ein relevantes 3D-Bild des Objekts (1) ausgewählt wird.

## Claims

1. Method for determining at least one relevant single image, wherein a plurality of optical single images are generated during a continuous optical measurement (3) of a dental subject (1) to be recorded, wherein an audio recording is generated during the optical measurement (3) by means of a microphone (4) and at least one speech sequence (12; 15; 28) spoken by a user (6) is recorded, **characterized in that** the relevant single image (7; 8; 24) is selected within a specified sequence duration between a start time (17, 18) and an end time of the speech sequence (12; 15; 28), wherein the relevant single image (7; 8; 24), which was recorded at a point in time in which a movement (3) of the dental camera (2) relative to the object to be recorded (1) was the least, is selected within the determined speech sequence.

2. Method according to Claim 1, **characterized in that**, during the processing of the audio recording, background noise (16) is filtered out by means of a filter unit (9) in order to improve the audio quality of the speech sequence (12; 15; 28).

3. Method according to Claim 2, **characterized in that** the filter unit (9) remains activated within a predetermined variable time period.

4. Method according to any one of Claims 1 to 3, **characterized in that** a maximum sequence duration (12; 15; 28) and/or a minimum sequence duration of the speech sequence is determined prior to carrying out the method.

5. Method according to any one of Claims 1 to 3, **characterized in that** the sequence duration of the speech sequence (12; 15; 28) corresponds to the duration of a coherent flow of words (13), wherein short interruptions (14) in the flow of words, which are shorter than a defined interruption duration, are ignored.

6. Method according to Claim 5, **characterized in that** the interruption duration is at most 2 seconds.

7. Method according to Claim 6, **characterized in that** only one relevant single image (7; 8; 24) is selected within a speech sequence (12; 15; 28).

8. Method according to any one of Claims 1 to 7, **characterized in that** the at least one speech sequence (12; 15; 28) contains status information and/or findings information (29; 30; 31), wherein the status information and/or the findings information (29; 30; 31) is converted from speech to text with the aid of speech recognition software.

9. Method according to Claim 1, **characterized in that** the identified status information and/or the findings information (29; 30; 31) is assigned to the relevant single image (7; 8; 24) of the respective speech sequence (12; 15; 28) in text form.

10. Method according to any one of Claims 1 to 9, **characterized in that**, during the optical measurement of the overall object (1), a plurality of relevant single images (7; 8; 24) are selected for the respective speech sequences (12; 15; 28), wherein the individual relevant single images (7; 8; 24) are displayed by means of a display device (21).

11. Method according to Claim 10, **characterized in that** the speech sequences (12; 15; 28) comprise anatomical position information, which is converted from speech to text with the aid of speech recognition software, wherein the anatomical position information is used to determine positions of the relevant single images (7; 8; 24) relative to the recorded object (1), wherein the relevant single images (7; 8; 24) at the determined positions are displayed relative to a virtual model (25) of the object (1) by means of the display device (21).

12. Method according to any one of Claims 1 to 11, **characterized in that** the selected relevant single images (7; 8; 24) are sorted into a number of categories of status information and/or findings information (29; 30; 31).

13. Method according to any one of Claims 1 to 12, **characterized in that** the optical measurement of the object (1) is performed by means of a video recording and the individual video images of this video recording constitute the selected single images (7; 8; 24), so that a relevant video image (7; 8; 24) is selected for each speech sequence (12; 15; 28).

14. Method according to any one of Claims 1 to 12, **characterized in that** the optical measurement of the object (1) is performed by means of an optical three-dimensional fringe projection method, wherein a plurality of individual 3D images of the object (1) are generated and combined to form a three-dimensional overall image, wherein these individual 3D images represent the single images to be selected, so that a relevant 3D image of the object (1) is selected for each speech sequence (12; 15; 28).

## Revendications

1. Procédé d'obtention d'au moins une image individuelle pertinente, plusieurs images optiques individuelles étant générées durant une mesure optique (3) en continu d'un objet dentaire (1) à capturer, un enregistrement audio étant généré au moyen d'un microphone (4) durant la mesure optique (3) et ce faisant au moins une séquence vocale (12 ; 15 ; 28) prononcée par un utilisateur (6) est enregistrée, **caractérisé en ce qu**'à l'intérieur d'une durée de séquence définie entre un instant de départ (17, 18) et un instant de fin de la séquence vocale (12 ; 15 ; 28), l'image individuelle pertinente (7 ; 8 ; 24) est sélectionnée, où, pendant la séquence vocale déterminée, l'image individuelle pertinente (7 ; 8 ; 24) qui a été capturée à un moment où un mouvement (3) de la caméra dentaire (2) par rapport à l'objet (1) à capturer était le plus faible est sélectionnée.

2. Procédé selon la revendication 1, **caractérisé en ce que**, lors du traitement de l'enregistrement audio, les bruits de fond (16) sont filtrés au moyen d'une unité de filtrage (9), pour améliorer la qualité audio de la séquence vocale (12 ; 15 ; 28).

3. Procédé selon la revendication 2, **caractérisé en ce que** l'unité de filtrage (9) reste activée durant une durée variable préalablement fixée.

4. Procédé selon l'une des revendications 1 à 3, **caractérisé en ce qu'**une durée de séquence (12 ; 15 ; 28) maximale et/ou une durée de séquence minimale de la séquence vocale sont fixées avant l'exécution du procédé.

5. Procédé selon l'une des revendications 1 à 3, **caractérisé en ce que** la durée de séquence de la séquence vocale (12 ; 15 ; 28) correspond à la durée d'un débit de paroles cohérent (13), de courtes interruptions (14) du débit de paroles qui sont inférieures à une durée d'interruption fixée étant ignorées.

6. Procédé selon la revendication 5, **caractérisé en ce que** la durée d'interruption maximale est de 2 secondes.

7. Procédé selon la revendication 6, **caractérisé en ce qu'**à l'intérieur d'une séquence vocale (12 ; 15 ; 28) une seule image individuelle pertinente (7 ; 8 ; 24) est sélectionnée.

8. Procédé selon l'une des revendications 1 à 7, **caractérisé en ce que** l'au moins une séquence vocale (12 ; 15 ; 28) contient une information d'état et/ou une information de résultat (29 ; 30 ; 31), l'information d'état et/ou l'information de résultat (29 ; 30 ; 31) étant transformées de parole en texte au moyen d'un logiciel de reconnaissance vocale.

9. Procédé selon la revendication 1, **caractérisé en ce que** l'information d'état et/ou l'information de résultat (29 ; 30 ; 31) reconnues sont associées sous forme de texte à l'image individuelle pertinente (7 ; 8 ; 24) de la séquence vocale (12 ; 15 ; 28) respective.

10. Procédé selon l'une des revendications 1 à 9, **caractérisé en ce que**, durant la mesure optique de l'objet (1) complet, plusieurs images individuelles pertinentes (7 ; 8 ; 24) sont sélectionnées pour les séquences vocales (12 ; 15 ; 28) respectives, les images individuelles pertinentes (7 ; 8 ; 24) étant affichées au moyen d'un dispositif d'affichage (21).

11. Procédé selon la revendication 10, **caractérisé en ce que** les séquences vocales (12 ; 15 ; 28) comprennent des indications de positions anatomiques, qui sont transformées de paroles en texte au moyen d'un logiciel de reconnaissance vocale, les positions des images individuelles pertinentes (7 ; 8 ; 24) étant déterminées par rapport à l'objet (1) moyennant l'utilisation des indications de positions anatomiques, les images individuelles pertinentes (7 ; 8 ; 24) étant représentées aux positions déterminées par rapport à un modèle virtuel (25) de l'objet (1) au moyen du dispositif d'affichage (21).

12. Procédé selon l'une des revendications 1 à 11, **caractérisé en ce que** les images individuelles pertinentes (7 ; 8 ; 24) sélectionnées sont triées en plusieurs catégories d'informations d'état et/ou d'informations de résultat (29 ; 30 ; 31).

13. Procédé selon l'une des revendications 1 à 12, **caractérisé en ce que** la mesure optique de l'objet (1) s'effectue au moyen d'un enregistrement vidéo et les images vidéos individuelles de cet enregistrement vidéo sont les images individuelles (7 ; 8 ; 24) sélectionnées, de sorte qu'une image vidéo pertinente (7 ; 8 ; 24) soit sélectionnée pour chaque séquence vocale (12 ; 15 ; 28).

14. Procédé selon l'une des revendications 1 à 12, **caractérisé en ce que** la mesure optique de l'objet (1) s'effectue au moyen d'un procédé de projection de franges tridimensionnel optique, plusieurs images 3D individuelles de l'objet (1) étant générées et assemblées en une image globale tridimensionnelle, ces images 3D individuelles représentant les images individuelles à sélectionner, de sorte qu'une image 3D pertinente de l'objet (1) soit sélectionnée pour chaque séquence vocale (12 ; 15 ; 28).
